(19) Europäisches Patentamt
European Patent Office
Office européen des brevets

(11) Publication number: 0 031 859 B1

# (12) EUROPEAN PATENT SPECIFICATION

(45) Date of publication of patent specification: 23.02.83

(51) Int. Cl.[3]: C 07 C 87/56, C 07 C 85/24

(21) Application number: 79303079.2

(22) Date of filing: 31.12.79

## (54) Production of 2,6-dimethyl aniline from 2,6-dimethyl cyclohexyl amine.

(43) Date of publication of application:
15.07.81 Bulletin 81/28

(45) Publication of the grant of the patent:
23.02.83 Bulletin 83/8

(84) Designated Contracting States:
AT BE CH DE FR GB IT LU NL SE

(56) References cited:
US - A - 3 361 818
US - A - 3 931 298

(73) Proprietor: ETHYL CORPORATION
330 South Fourth Street P.O. Box 2189
Richmond Virginia 23219 (US)

(72) Inventor: Shin, Kju Hi
2645 Hickory Court
Bloomfield Hills Michigan (US)
Inventor: Wollensak, John Charles
666 Weybridge
Bloomfield Hills Michigan (US)

(74) Representative: Baillie, Iain Cameron et al,
c/o Ladas & Parry Isartorplatz 5
D-8000 München 2 (DE)

EP 0 031 859 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Production of 2,6-dimethyl aniline from 2,6-dimethyl cyclohexyl amine

This invention relates to the production of 2,6-dimethyl aniline. More particularly the invention comprises an improved process for generating this compound from cyclohexyl amines. It is known to produce aromatic amines by contacting a cyclohexyl amine with a Group VIII metal catalyst preferably in the presence of ammonia and hydrogen. However, when this technique is applied to producing 2,6-dimethyl aniline, it is found that formation of the desired product is very slow. The present invention provides substantial improvement in the rate of formation. It comprises heating a mixture of 2,6-dimethylcyclohexylamine and a phenol in contact with a palladium catalyst, maintaining the reactants in a liquid phase and at an elevated temperature of about 200 to 400°C.

Various methods have been used in the past to prepare aromatic amines. According to one method, an aromatic compound is nitrated to form a nitroaromatic which is then hydrogenated to the corresponding aromatic amine. This method is used commercially to make aniline from benzene.

Van Verth *et al,* U.S. 3,219,702, describes a process in which a cyclohexanone is reacted with ammonia in the presence of a hydrogen acceptor (e.g. nitrobenzene) and a dehydrogenation catalyst.

Wilder *et al,* U.S. 3,219,704, describes a similar process in which the hydrogen acceptor is eliminated and the amount of cyclohexanone is at least equivalent to the amount of ammonia used.

Barker, U.S. 3,272,865, describes the production of anilines by reaction of phenols with ammonia in contact with a silica-alumina, titania-alumina, zirconia-alumina, phosphoric acid or tungsten oxide catalyst. The process is similar to that described by Neuroessen, U.S. 1,935,209, and Lowy *et al,* U.S. 1,449,423.

Barker, U.S. 3,361,818, describes the production of aromatic amines by passing cyclohexyl amine through a Group VIII metal catalyst. Preferably hydrogen and ammonia are included.

Barker, U.S. 3,442,950, describes the reaction of a cyclohexanol with an aminating agent (e.g. ammonia) in contact with a metal catalyst to form the corresponding aromatic amine. If cyclohexanone is present, hydrogen is added initially in an amount at least equivalent to the amount of cyclohexanone.

Wollensak, U.S. 3,931,298, describes the production of aromatic amines by the reaction of a phenol with ammonia in contact with a Group VIII metal catalyst and a promoter amount of a cyclohexanone.

Other processes for making aromatic amines are taught by Ballard, U.S. 2,413,598; Vogt, U.S. 2,013,873; Groggins, "Unit Processes in Organic Synthesis," 5th Ed.; and Houben-Weyl, "Methoden der Organische Chemi," Vol. 11/1, pp. 117—122.

According to the present process, 2,6-dimethyl aniline is formed from 2,6-dicyclohexyl amine by reaction with a hydroxy aromatic at elevated temperatures, in contact with a palladium catalyst. The 2,6-dimethylcyclohexyl amine can be initially made by reacting 2,6-dimethylphenol with hydrogen and ammonia either sequentially or concurrently in contact with any of a variety of hydrogenation catalysts, e.g. Ni.

A preferred embodiment of the invention is a process for making 2,6-dimethyl aniline, said process comprising heating a mixture of 1 to 10 mole parts of a 2,6-dimethylcyclohexyl amine and 1 to 10 mole parts of a phenol at a temperature of 200 to 400°C. in contact with a palladium catalyst and under sufficient pressure to maintain the reactants in a liquid phase and recovering said aromatic amine from the resultant mixture.

The phenol used in the process can be any phenol which does not adversely effect the reaction. It is not necessary that the phenol correspond in structure with the cyclohexyl amine. The phenol may be unsubstituted or substituted with such groups as alkyl, cycloalkyl, aryl, aralkyl, haol, alkoxy and the like. Some examples of such phenols are:

phenol
*p*-cresol
*o*-cresol
2,6,-dimethylphenol
2,6-diethylphenol
2-methyl-6-isopropylphenol
2,6-diisopropylphenol
2-methyl-6-*tert*-butylphenol
2,6-di-*tert*-butylphenol
2-*sec*-butylphenol
2,6-di-*sec*-butylphenol
2,4-dimethylphenol
4-*sec*-octylphenol
2-*sec*-dodecyl-4-methylphenol
2-*sec*-eicosylphenol
4-cyclohexylphenol
4-phenylphenol

1-naphthol
2-naphthol
2-(α,α-dimethylbenzyl)phenol
2,6-di-(α-methylbenzyl)phenol
4-(α,α-dimethylbenzyl)phenol
2,4-di-(α,α-dimethylbenzyl)phenol
4-chlorophenol
2,4-dichlorophenol
2,4,6-trichlorophenol
4-bromophenol
2,6-dibromophenol
4-fluorophenol
4-iodophenol
2,6-dichlorophenol
4-methyoxyphenol
2-ethyoxyphenol
4-dodecoxyphenol

and the like.

In a more preferred embodiment the phenol corresponds in structure with the cyclohexyl amine. In other words the preferred phenol is 2,6-dimethylphenol.

The reaction can be carried out by merely mixing the 2,6-dimethylcyclohexyl amine with the phenol and heating the mixture at 200 to 400°C. in contact with the catalyst. Preferably the palladium is on a suitable catalyst support such as alumina, silica-alumina, magnesia, zirconia, or the like. The preferred catalyst support is charcoal. The amount of catalyst can vary over a wide range. Reaction rate is dependent on the amount of catalyst used. A useful range is from 0.0001 to 0.01 mole parts of palladium excluding support per mole part of reaction mixture. A preferred range is 0.0005 to 0.001 mole of palladium per mole of reaction mixture.

The amount of each reactant can vary over a wide range. A useful range is from 1 to 10 mole parts of 2,6-dimethylcyclohexyl amine for each 1 to 10 mole parts of phenolic reactant. More preferably, the process is carried out with 1 to 2 mole parts of a 2,6-dimethylcyclohexyl amine for each 1 to 2 mole parts of a phenol. Most preferably equal mole amounts are used.

The reaction is effected by heating the mixture to 200 to 400°C. A more preferred temperature range is 225 to 300°C. The reaction is preferably carried out at autogenous pressure in a closed vessel such as an autoclave to maintain the reactants in the liquid phase. The reaction is conducted for a time sufficient to convert a substantial amount of the cyclohexyl amine to aromatic amine. A useful reaction time is from 30 minutes to 8 hours. Generally, good results are achieved in 1 to 4 hours.

The reaction will proceed without the necessity of adding hydrogen or ammonia to the autoclave. It is highly preferred that the process be conducted without adding hydrogen or ammonia. Preferably an inert atmosphere such as nitrogen is placed over the reactants at the start of the reaction.

The 2,6-dimethylcyclohexyl amine is believed to be converted to the corresponding aromatic amine according to the following equation:

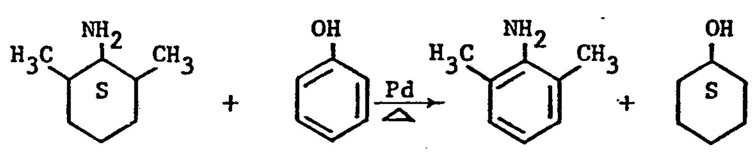


Some cyclohexanone forms. The aromatic amine can be readily recovered from the mixture by distillation. When 2,6-dimethylphenol is used the 2,6-dimethylcyclohexanol and cyclohexanone (referred to collectively herein as "reduced products") which form are not waste products but can be recycled to the process after being converted to the corresponding 2,6-dimethylcyclohexyl amine. This can be accomplished by known methods such as by reacting the reduced products with ammonia in the presence of a dehydration catalyst such as activated alumina at temperatures of 200 to 500°C. Alternatively, the reduced products can be reacted with a mixture of hydrogen and ammonia in the presence of a hydrogenation catalyst at 200 to 500°C. to form 2,6-dimethylcyclohexyl amine. The hydrogenation catalyst need not be palladium but can be any of the metal catalysts known to catalyze hydrogenation such as the transition metal catalysts, especially the less costly catalysts made from metals of the First Transition Series, viz., V, Cr, Mn, Fe, Co, Ni, Cu, and the like. Preferably the hydrogenation catalyst is a Groupe VIII metal catalyst. When a metal hydrogenation catalyst is used to convert the reduced products to 2,6-dimethylcyclohexyl amine it is preferably removed from the cyclohexyl amine product and replaced with the preferred palladium catalyst for the reaction of the 2,6-dimethylcyclohexyl amine with the phenol in the manner previously described.

The method according to which the process is conducted is illustrated by the following examples.

Example 1

In an autoclave was placed 60 grams of a mixture consisting essentially of 52 grams of 2,6-dimethylcyclohexyl amine and 8 grams of 2,6-dimethyl aniline. To this was added 13 grams of 5 percent palladium on charcoal catalyst and 90 grams of 2,6-dimethylphenol. The autoclave was sealed and heated to 250°C. and maintained at that temperature for 2 hours. It was then cooled and analyzed by vapor phase chromatography (VPC). It contained 48.5 grams of 2,6-dimethyl aniline, recoverable by distillation.

Barker, U.S. 3,361,818, discloses a process for converting cyclohexyl amines to anilines by merely heating the cyclohexyl amine to 180 to 500°C. in contact with a catalyst such as palladium. That process appears to work well in converting cyclohexyl amine to aniline. However, it is not very effective in converting 2,6-dimethylcyclohexylamine to 2,6-dimethylaniline.

Comparative tests were conducted which show the significant effect of the phenol on the reaction of 2,6-dimethylcyclohexylamine in contact with a palladium catalyst. The first test was conducted by heating 2,6-dimethylcyclohexylamine in contact with a palladium catalyst without adding a phenol. The following example describes this test.

Example 2

In an autoclave was placed 87 grams of 2,6-dimethylcyclohexylamine and 14.6 grams of wet 5 percent Pd on charcoal. The autoclave was flushed with hydrogen. It was then pressurized with hydrogen to 1,000 psig (70.3 kg/sq. cm.) and heated to 50°C. as a standard method of activating the Pd catalyst. The autoclave was cooled and vented and 18 grams of ammonia was added. It was sealed and heated to 300°C. considered the "start" of the reaction. It was necessary to vent 8 grams of ammonia to keep the pressure within the limit of the autoclave. The autoclave was maintained at 300°C. for three hours with samples taken at the start and hourly. The samples analyzed as follows:

Area Percent VPC Analysis of Reaction Mixture

| | A | B | C | D | E | 2,6-dimethyl-aniline |
|---|---|---|---|---|---|---|
| initial | 6.2 | 1.9 | — | 88.5 | — | 1.0 |
| "start" | 3.3 | 1.8 | — | 74.3 | 0.04 | 9.0 |
| 1 hour | — | 10.6 | 1.5 | 70.4 | 0.3 | 15.3 |
| 2 hours | — | 18.8 | 1.9 | 60.3 | 0.2 | 17.8 |
| 3 hours | — | 24.1 | 3.0 | 51.7 | 0.5 | 20.0 |

A = water; B = 2,6-dimethylcyclohexane; C = *m*-xylene; D = 2,6-dimethylcyclohexylamine; E = 2,6-dimethylphenol.

In this experiment carried out without adding 2,6-dimethylphenol, the formation of 2,6-dimethylaniline was very slow. The major products after three hours were (B) 2,6-dimethylcyclohexane and (C) *m*-xylene.

The following example shows the effect of including 2,6-dimethylphenol in the above reaction.

Example 3

In an autoclave was placed 40 grams of 2,6-dimethylcyclohexylamine, 38.5 grams 2,6-dimethylphenol and 13.4 grams wet 5 percent Pd on charcoal. The autoclave was flushed with hydrogen and then pressurized with hydrogen to 1,000 psig (70.3 kg./sq. cm.) and heated to 50°C. to activate the catalyst. The autoclave was cooled and vented and 9 grams of ammonia added. The autoclave was heated to 300°C., which was considered the "start" of the reaction. Samples were taken at the start and hourly for VPC analysis. The following table shows the results of the above reaction:

Area Percent VPC Analysis of Reaction Mixture

| | A | B | C | D | E | F | G | H | 2,6-dimethyl aniline |
|---|---|---|---|---|---|---|---|---|---|
| initial | 2.7 | 0 | 0 | 42.2 | 53.1 | 0 | 0 | 1.2 | 0.8 |
| "start" | — | 0 | 3.2 | 33.5 | 6.8 | 1.7 | 0.8 | 0.4 | 52.2 |
| 1 hour | — | 0 | 16.1 | 5.9 | 3.6 | 10.5 | 4.3 | 0.9 | 58.6 |
| 2 hours | — | 0 | 27.5 | 2.2 | 3.0 | 12.1 | 4.6 | 0.8 | 46.9 |
| 3 hours | — | 0 | 28.4 | 1.5 | 2.8 | 12.0 | 4.5 | 0.7 | 48.3 |

A = water; C = *m*-xylene; D = 2,6-dimethylcyclohexylamine; E = 2,6-dimethylphenol; F, G and H = unknowns.

The above results show that when the same reaction as in Example 2 is carried out in the presence of 2,6-dimethylphenol, the 2,6-dimethylaniline forms very rapidly giving a reaction mixture analyzing over 50 percent 2,6-dimethylaniline.

**Claims**

1. A process for producing 2,6-dimethyl aniline by heating 2,6-dimethylcyclohexyl amine in the presence of a palladium catalyst characterized by heating 1 to 10 mole parts of 2,6-dimethylcyclohexyl amine with 1 to 10 mole parts of a phenol at a temperature of 200° to 400°C. in contact with a palladium catalyst and under sufficient pressure to maintain the reactants in a liquid phase and then recovering 2,6-dimethyl aniline from the resultant mixture.

2. A process according to claim 1 characterized in that the phenol is 2,6-dimethylphenol.

**Revendications**

1. Procédé de préparation de 2,6-diméthyl-aniline par chauffage de 2,6-diméthylcyclohexylamine en présence d'un catalyseur au palladium, caractérisé en ce que l'on chauffe 1 à 10 parties en mole de 2,6-diméthylcyclohexyl-amine avec 1 à 10 parties en mole de phénol à une température de 200 à 400°C au contact d'un catalyseur au palladium et sous une pression suffisante pour maintenir les réactifs en phase liquide, et en ce que l'on récupère la 2,6-diméthyl-aniline à partir du mélange résultant.

2. Procédé selon la revendication 1, caractérisé en ce que le phénol utilisé est le 2,6-diméthylphénol.

**Patentansprüche**

1. Verfahren zum Erzeugen von 2,6-Dimethylanilin durch Erhitzen von 2,6-Dimethylcyclohexylamin in Gegenwart eines Palladiumkatalysators, dadurch gekennzeichnet, daß 1 bis 10 Molteile 2,6-Dimethylcyclohexyl mit 1 bis 10 Molteilen eines Phenols bei einer Temperatur von 200 bis 400°C in Berührung mit einem Palladiumkatalysator und unter einem Druck erhitzt werden, der genügt, um die Reaktionspartner in der flüssigen Phase zu halten, und danach aus dem so erhaltenen Gemisch 2,6-Dimethylanilin gewonnen wird.

2. Verfahren nach Anspruch 1, dadurch gekennzeichnet, daß das Phenol 2,6-Dimethylphenol ist.